(19) **Europäisches Patentamt**

**European Patent Office**

**Office européen des brevets**

(11) **EP 1 069 877 B1**

(12) **EUROPÄISCHE PATENTSCHRIFT**

(45) Veröffentlichungstag und Bekanntmachung des Hinweises auf die Patenterteilung:
**11.08.2004  Patentblatt 2004/33**

(51) Int Cl.$^7$: **A61F 9/008**

(21) Anmeldenummer: **00902651.9**

(22) Anmeldetag: **02.02.2000**

(86) Internationale Anmeldenummer:
**PCT/EP2000/000827**

(87) Internationale Veröffentlichungsnummer:
**WO 2000/045759 (10.08.2000 Gazette 2000/32)**

(54) **VORRICHTUNG FÜR DIE PHOTOREFRAKTIVE HORNHAUTCHIRURGIE BEI SEHFEHLERN HÖHERER ORDNUNG**

DEVICE FOR PHOTOREFRACTIVE CORNEA SURGERY IN HIGHER-ORDER VISUAL DISORDERS

SYSTEME UTILISE DANS LE CADRE DE LA CHIRURGIE PHOTOREFRACTIVE DE LA CORNEE DANS LE CAS DE TROUBLES DE LA VISION D'ORDRE SUPERIEUR

(84) Benannte Vertragsstaaten:
**AT BE CH CY DE DK ES FI FR GB GR IE IT LI LU MC NL PT SE**

(30) Priorität: **05.02.1999  DE 19904753**

(43) Veröffentlichungstag der Anmeldung:
**24.01.2001  Patentblatt 2001/04**

(73) Patentinhaber: **Wavelight Laser Technologie AG 91058 Erlangen (DE)**

(72) Erfinder:
- **SEILER, Theo D-95239 Zell (DE)**
- **MROCHEN, Michael D-01219 Dresden (DE)**
- **KAEMMERER, Maik D-01169 Dresden (DE)**

(74) Vertreter: **von Hellfeld, Axel, Dr. Dipl.-Phys. et al Wuesthoff & Wuesthoff Patent- und Rechtsanwälte Schweigerstrasse 2 81541 München (DE)**

(56) Entgegenhaltungen:
**WO-A-99/27334          DE-C- 19 702 335**

- **LOPEZ-GIL, N.: "Generation of third-order spherical and coma aberrations by use of radially symmetrical fourth-order lenses." JOURNAL OF THE OPTICAL SOCIETY OF AMERICA, Bd. 15, Nr. 9, September 1998 (1998-09), Seiten 2563-2571, XP002138673 usa**

**Beschreibung**

Die Erfindung betrifft eine Vorrichtung für die photorefraktive Hornhautchirurgie des Auges zur Korrektur von Sehfehlern höherer Ordnung.

Die photorefraktive Keratektomie (englisch: Photorefractive Keratectomy) ist bisher ein weitgehend etabliertes Verfahren zur Korrektur von Fehlsichtigkeit niederer Ordnung, also zum Beispiel von Myopie, Hyperopie, Astigmatismus, myopem Astigmatismus und hyperopem Astigmatismus. Der Begriff "phororefraktive Keratektomie (PRK)" wird üblicherweise dahingehend verstanden, daß damit nur ein Eingriff an der Hornhautoberfläche gemeint ist, nachdem das sog. Hornhautepithel entfernt ist. Nach Entfernung des Epithels liegt die Bowman-Membran bzw. das Hornhautstroma frei und kann mit einem Laser abgetragen werden. Von der PRK im allgemeinen unterschieden wird das LASIK-Verfahren (Laser In Situ Keratomileusis). Beim LASIK-Verfahren wird zunächst mit einem sog. Mikrokeratom ein ca. 100 μm bis 200 μm dickes Hornhautscheibchen (sog. "Flap") mit einem Durchmesser von 8 bis 10 mm bis auf einen geringen, als "Scharnier" dienenden Rest abgeschnitten. Dieses Scheibchen (Flap) wird zur Seite geklappt und danach erfolgt die Ablation (Entfernung) von Material mittels Laserstrahlung direkt im Stroma, also nicht an der Hornhautoberfläche. Nach der Laserbehandlung wird der Deckel wieder an seinen ursprünglichen Platz zurückgeklappt und es erfolgt in der Regel eine relativ schnelle Heilung.

Die nachfolgend beschriebene Erfindung eignet sich sowohl für die vorstehend erläuterte PRK als auch, insbesondere, für die LASIK-Technik.

Bei der PRK und bei LASIK wird Material der Hornhaut abgetragen. Der Abtrag ist eine Funktion der auf die Hornhaut auftreffenden Energiedichte (Energie pro Flächeneinheit) des Laserstrahls. Es sind unterschiedliche Techniken für die Strahlformung und Strahlführung bekannt, so zum Beispiel die sogenannte Schlitz-Abtastung (slit scanning), bei der die Strahlung mittels eines bewegten Schlitzes über den zu bearbeitenden Bereich geführt wird, das sogenannte Fleck-Abtasten (scanning-spot), bei dem ein Strahlungsfleck mit sehr geringen Abmessungen über das abzutragende Gebiet geführt wird, und auch die sogenannte Vollabtragung (full-ablation oder widefield ablation), bei der die Strahlung großflächig über den gesamten abzutragenden Bereich eingestrahlt wird und wobei die Energiedichte sich über das Strahlprofil ändert, um den gewünschten Abtrag der Hornhaut zu erreichen. Der Stand der Technik kennt für die genannten Strahl-Führungen jeweils geeignete Algorithmen zum Steuern der Strahlung, um die Hornhaut so abzutragen, daß die Cornea schließlich den gewünschten Krümmungsradius erhält.

Das vorstehend bereits erwähnte "Fleck-Abtasten" (scanning- spot) verwendet einen auf einen relativ kleinen Durchmesser (0,1-2mm) fokussierten Laserstrahl, der mittels einer Strahlführungseinrichtung auf verschiedene Stellen der Hornhaut gerichtet und durch einen sogenannten Abtaster (scanner) sukzessive so bewegt wird, dass letztlich der gewünschte Abtrag von der Cornea erreicht wird. Die Abtragung erfolgt also gemäß einem sogenannten Ablationsprofil. Bei der PRK und LASIK sind insbesondere sogenannte galvanometrische Abtaster (Scanner) verwendbar (vgl. Aufsatz G.F. Marshall in LASER FOCUS WORLD, Juni 1994, S. 57). Es sind inzwischen auch andere Scan-Techniken bekannt für die Führung des Laserstrahls.

Nach dem Stand der Technik werden zur Zeit die genannten Fehlsichtigkeiten niederer Ordnung (z.B. Myopie, Hyeropie, Astigmatismus) nach den sogenannten Refraktionsdaten des Patientenauges durchgeführt, d.h. der für das Patientenauge gemessene Dioptrie-Wert bestimmt das Ablationsprofil, gemäß dem Material von der Hornhaut abgetragen (ablatiert) wird (vgl. T. Seiler und J. Wollensak in LASERS AND LIGHT IN OPTHALMOLOGY, Vol. 5, Nr. 4, S. 199-203, 1993). Gemäß diesem Stand der Technik wird also für ein gegebenes Patientenauge mit einem bestimmten Dioptrie-Wert die Laserstrahlung so über die Hornhaut (Cornea) geführt, dass ein vorgebenes Ablationsprofil abgetragen wird, zum Beispiel entsprechend einer Parabel bei einer Myopiekorrektur. Mit anderen Worten: das Ablationsprofil ist nur gemäß dem Dioptrie-Wert an das individuelle Auge angepasst nicht aber gemäß lokalen Unregelmäßigkeiten des optischen Systems "Auge".

Auch der Aufsatz von J.K. Shimmick, W.B. Telfair et al. in JOURNAL OF REFRATIVE SURGERY, Vol. 13, Mai/Juni 1997, S. 235-245, beschreibt die Korrektur von Sehfehlern niederer Ordnung mittels photorefraktiver Keratektomie, wobei die Photoablationsprofile theoretischen Parabelformen entsprechen. Es wurde darüber hinaus dort nur vorgeschlagen, einige empirische Korrekturfaktoren in das Ablationsprofil einzufügen, die der Wechselwirkung zwischen Laser und Gewebe Rechnung tragen, um im Ergebnis eine paraboloidförmige Abtragung auf dem Auge zu erreichen.

Ein besonderes Problem bei der photorefraktiven Keratektomie und LASIK ist die relative Positionierung von Laserstrahl und Auge. Der Stand der Technik kennt verschiedene Verfahren hierfür, so zum Beispiel sogenannte "Eyetracker", d.h. Einrichtungen, die Bewegungen des Auges ermitteln, um dann den für die Ablation verwendeten Laserstrahl entsprechend den Augenbewegungen zu steuern (nachzuführen). Den Stand der Technik hierzu beschreibt zum Beispiel die DE 197 02 335 C1.

Wie vorstehend erwähnt ist, sind die Verfahren der photorefraktiven Hornhautchirurgie des Standes der Technik zur Korrektur von Fehlsichtigkeit niederer Ordnung im wesentlichen "Pauschalverfahren" in dem Sinne, dass die Korrektur auf den (pauschalen) Dioptrie-Wert des Auges abstellt. Die Korrektur derartiger Fehlsichtigkeit niederer Ordnung kann zum Beispiel mit sphärischen oder astigmatischen Linsen oder auch eben mit einer photorefraktiven Korrektur der Hornhaut erfolgen.

Allerdings wird die optische Abbildung im Auge nicht nur durch die genannten Fehlsichtigkeiten niederer Ordnung beeinträchtigt, sondern auch durch sogenannte Bildfehler höherer Ordnung. Solche Bildfehler höherer Ordnung treten insbesondere auf nach operativen Eingriffen an der Hornhaut und innerhalb des Auges (Katarakt-Operationen). Solche optischen Aberrationenen könne die Ursache dafür sein, dass trotz einer ärztlichen Korrektur eines Fehlers niederer Ordnung die volle Sehschärfe (Visus) nicht erreicht wird. P. Mierdel, H.-E. Krinke, W. Wigand, M. Kaemmerer und T. Seiler beschreiben in DER OPHTALMOLOGE, Nr. 6, 1997, S.441 eine Messanordnung zur Bestimmung der Aberration des menschlichen Auges. Mit einer solchen Messanordnung können Aberrationen (Abbildungsfehler) für monochromatisches Licht gemessen werden, und zwar nicht nur durch die Hornhaut bedingte Aberrationen, sondern es können die vom gesamten okularen Abbildungsystem des Auges verursachten Abbildungsfehler gemessen werden, und zwar ortsabhängig, d.h. mit einer bestimmten Auflösung kann für gegebene Orte innerhalb der Pupille des Auges bestimmt werden, wie groß an dieser Stelle der Abbildungsfehler des gesamten optischen Systems des zu korrigierenden Auges ist. Derartige Abbildungsfehler des Auges werden in der vorstehend zitierten Arbeit von P. Mierdel et al. als sogenannte Wellenfrontaberration mathematisch beschrieben. Man versteht unter einer Wellenfrontaberration den räumlichen Verlauf des Abstands zwischen der realen Lichtwellenfront eines zentralen Lichtpunktes und einer Referenzfläche, wie z. B. ihrer idealen, kugelförmigen Gestalt. Als räumliches Bezugssystem dient also z. B. die Kugeloberfläche der idealen Wellenfront. Es ist auch als solches im Stand der Technik bekannt, als Bezugssystem für die Aberrationsmessung eine Ebene zu wählen, wenn die zu vermessende ideale Wellenfront eben ist.

Das Messprinzip gemäß der genannten Arbeit von P. Mierdel, T. Seiler et al. wird auch bei Verwirklichung der vorliegenden Erfindung als Ausgangsschritt eingesetzt. Es beinhaltet im wesentlichen, daß ein Parallelstrahlbündel hinreichenden Durchmessers durch eine Lochmaske in getrennte parallele Einzelstrahlen aufgeteilt wird. Diese Einzelstrahlen durchlaufen eine Sammellinse (sogenannte Aberroskoplinse) und werden dadurch beim emmetropen Auge in einem bestimmten Abstand vor der Retina fokussiert. Die Folge sind gut sichtbare Projektionen der Maskenlöcher auf der Retina. Dieses retinale Lichtpunktmuster wird nach dem Prinzip der indirekten Ophtalmoskopie auf die Sensorfläche einer CCD-Videocamera abgebildet. Im aberrationsfreien idealen Auge ist das abgebildete Lichtpunktmuster unverzerrt und entspricht genau dem Lochmaskenmuster. Ist aber eine Aberration gegeben, kommt es zu individuellen Verschiebungen jedes Musterpunktes weil jeder Einzelstrahl einen bestimmten Hornhaut- bzw. Pupillenbereich durchläuft und gemäß der irregulären optischen Wirkung eine Abweichung vom idealen Verlauf erfährt. Aus den retinalen Musterpunktverschiebungen wird schließlich die Wellenfrontaberration mit einem Näherungsverfahren als Ortsfunktion über der Pupillenfläche ermittelt. Der genannte Stand der Technik beschreibt auch die mathematische Darstellung dieser Wellenfrontaberretion in Form eines sogenannten "Wellenfrontaberrationsgebirges". Dieses "Wellenfrontaberrationsgebirge" gibt über jedem Pupillenort (x-y Koordinaten) einen Wert für die Wellenfrontaberration W(x,y) an, der dann als Höhe über den x-y Koordinaten aufgetragen ist. Je höher das "Gebirge" um so größer sind die Abbildungsverzerrungen im Auge an dem jeweiligen Pupillenort. Für jeden einfallenden Lichtstrahl besteht in erster Näherung eine Proportionalität zwischen der gemessenen Abweichung des entsprechenden retinalen Lichtpunktes von seiner idealen Position und der Steilheit des "Wellenfrontaberrationsgebirges". Somit kann daraus die Wellenfrontaberration als Ortsfunktion, bezogen auf einen willkürlichen Referenzwert auf der optischen Achse des Systems, bestimmt werden. Ideale, im Regelfall unverzerrte Lichtpunktpositionen auf der Retina, die den Referenzwert liefern können, sind zum Beispiel vier zentrale Punkte mit geringem gegenseitigen Abstand. Solche Punkte repräsentieren eine zentrale Hornhaut-Pupillen-Zone von etwa 1 bis 2 mm Durchmesser, die Erfahrungsgemäß als weitgehend frei von Bildfehlern höherer Ordnung angenommen werden kann.

Das "Wellenfrontaberrationsgebirge" kann in verschiedener Weise mathematisch mit Hilfe eines geschlossenen Ausdruckes (einer Funktion) dargestellt werden. In Betracht kommen z. B. Approximationen in Form einer Summe von Taylor- oder auch insbesondere Zernike-Polynomen. Die Zernike-Polynome haben den Vorteil, daß ihre Koeffizenten einen direkten Bezug zu den allgemein bekannten Bildfehlern (Öffnungsfehler, Koma, Astigmatismus, Verzeichnung) haben. Die Zernike-Polynome sind ein Satz vollständig orthogonaler Funktionen. In einem Aufsatz von J. Liang, B. Grimm, S. Goelz und J. F. Bille, "Objective Measurement of Wave Aberrations of the Human Eye with the use of a Hartmann-Shack Wave-Front Sensor, *Optical Society of America*, 11(7):1949-1957, Juli 1994, wird gezeigt, wie die Wellenfront (bzw. Wellenfrontaberration) aus den Gitterpunktverschiebungen berechnet werden kann. Aus der Bestimmung der Ableitungsfunktion der Wellenfront läßt sich so die eigentliche Wellenfront ermitteln. Die Wellenfront ergibt sich als Lösung eines Gleichungssystems. Auch der Aufsatz von H. C. Howland und B. Howland, "A Subjective Method for the Measurement of Monochromatic Aberrations of the Eye", *Journal of the Optical Society of America*, 67(11): 1508-1518, November 1977, beschreibt ein Verfahren zum Bestimmen der monochromatischen Aberration und die Ermittlung der ersten fünfzehn Taylor-Koeffizienten. Auf diesen Stand der Technik kann zurückgegriffen werden.

Eine Messung der Aberration und der retinalen Bildqualität des menschlichen Auges wird auch in folgendem Aufsatz beschrieben: "Aberrations and retinal image quality of the normal human eye", Junzhong Liang and David R.

Williams, Journal Optical Society America A, Vol. 14, No. 11, November 1997, S. 2873-2883.

In der WO 99/27334 (veröffentlicht nach dem Prioritätstag der vorliegenden Anmeldung) wird die Wellenfrontaberration des Auges gemessen und für die nachfolgende Ablation verwendet.

Der Stand der Technik kennt auch schon den Versuch, Ablationsprofile (Abtragsprofile) individuell ortsabhängig für ein zu korrigierendes Auge zu ermitteln, und zwar basierend auf sogenannten topographischen Messungen der Hornhautoberfläche, vgl. C.E. Martinez, R.A. Applegate et al. in ARCH OPHTHALMOL/Vol. 116, Aug. 1998, S. 1053-1062. Derartige Topographien der Hornhautoberfläche liefern jedoch nur Daten über die Hornhautkrümmung, d. h. Höhendaten an jedem Punkt der Hornhautoberfläche. Aus diesen Daten lassen sich zwar Aberrationen berechnen, jedoch liefern diese Daten nur Fehler höherer Ordnung an der Hornhautoberfläche und nicht Aberrationswerte für das gesamte optische System "Auge". Das Auflösungsvermögen des Auges (Visus) wird jedoch nicht nur durch die Hornhautoberfläche, sondern durch das gesamte optische System des zu korrigierenden Auges bestimmt (z.B. auch die Augenlinse), so dass auch in soweit eine Verbesserung wünschenswert ist.

Der Erfindung liegt die Aufgabe zugrunde, eine Vorrichtung für die photorefraktive Keratektomie bereitzustellen, mit der Fehlsichtigkeiten höherer Ordnung mit verbesserten Ergebnissen behandelt werden können.

Die erfindungsgemäße Vorrichtung zur Lösung dieser Aufgabe ist im Patentanspruch 1 beschrieben.

Der Erfindung liegt die Erkenntnis zugrunde, dass es im Anschluss an die PRK oder LASIK durch den Heilungsprozess zu einer Änderung der optischen Eigenschaften des Auges kommt und dass diese Änderung bei der Bestimmung des Photoablationsprofils berücksichtigt werden sollte.

In den abhängigen Ansprüche sind bevorzugte Ausgestaltungen der Erfindung beschrieben.

Die Vermessung der optischen Eigenschaften des zu behandelnden Auges kann auch gemäß einem weiteren Verfahren durchgeführt werden: Bei diesem Verfahren bzw. einer dieses Verfahren durchführenden Vorrichtung, das bzw. die weiter unten näher beschrieben wird, wird das Ablationsprofil direkt aus der Projektion von Punkten auf die Hornhaut und die Netzhaut berechnet. "Projektion" bedeutet hier, daß ein Lichtstrahl geringen Durchmessers auf die Hornhaut gerichtet wird, dort den genannten Punkt erzeugt und von der Hornhaut auf die Netzhaut gelangt, wo er einen weiteren Punkt erzeugt. Die Punkte sind Lichtflecken. Aus einer Abweichung der Position des Lichtflecks auf der Netzhaut von einer Sollposition (die Sollposition entspricht einem aberrationsfreien Auge) läßt sich (siehe unten) auf eine Änderung der Krümmung der Hornhautoberfläche schließen, was letztlich eine Aussage über die Ableitungsfunktion (im mathematischen Sinn) des gesuchten Ablationsprofils bedeutet. Wird dieses Verfahren mit einer ausreichenden Anzahl von Lichtstrahlen durchgeführt, die an unterschiedlichen Stellen des Auges eingestrahlt werden, lässt sich über die gesamte interessierende Fläche des Auges die Ableitungsfunktion des Ablationsprofils ermitteln und hieraus dann mathematisch das Ablationsprofil selbst berechnen. Die Erfindung beinhaltet auch die apperativen Mittel zur Durchführung dieses Verfahrens, also insbesondere die Mittel zur Einstrahlung ausgewählter Lichtstrahlen mit definierten Positionen und Einfallswinkeln, die Mittel zur Messung einer Verschiebung des Lichtstrahls auf der Netzhaut in bezug auf die Sollposition und die entsprechend programmierten Rechenmittel zum Ermitteln von Photoablationsprofilen aus diesen Messungen von Lichtstrahl-Positionen auf der Netzhaut.

Nachfolgend wird ein Ausführungsbeispiel der Erfindung an Hand der Zeichnung näher erläutert. Es zeigt:

Figur 1    schematisch die Wellenfrontaberration;

Figur 2    schematisch ein Aberroskop zum Messen der Wellenfrontaberration des gesamten optischen Systems eines zu behandelnden Auges, und

Figur 3    schematisch eine Meß- und Steueranordnung zum Durchführen einer photorefraktiven Keratektomie des Auges, Mitteln zum Ableiten eines Photoablationsprofils und Mitteln zum Steuern der Laserstrahlung.

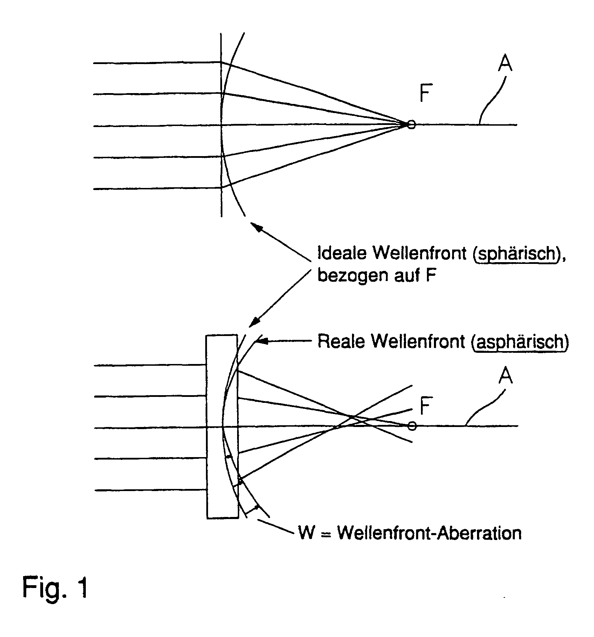

Figur 1 zeigt schematisch die oben bereits erläuterte Wellenfrontaberration eines Auges, d. h. die Abweichung der realen, asphärischen Wellenfront von der idealen Wellenfront. A ist die optische Achse des Systems und F der Brennpunkt, letzteres hier auch der gedachte Ausgangspunkt der Strahlung im Falle einer idealen Wellenfront.

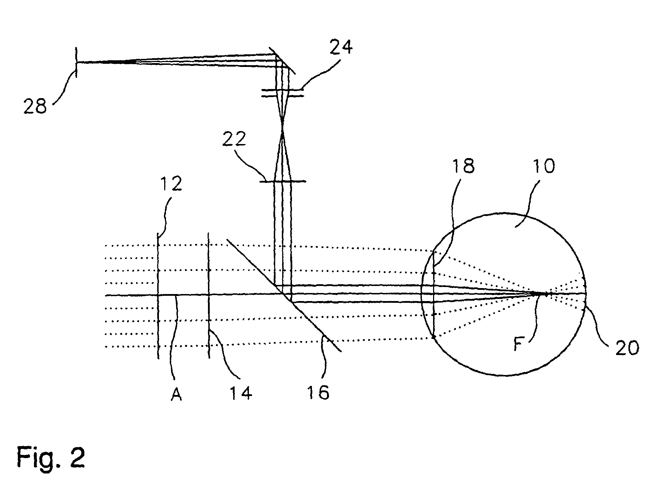

Figur 2 zeigt schematisch das optische Schema eines Video-Aberroskops zur Messung der Wellenfrontaberration eines Auges 10. Das grüne Licht eines HeNe-Lasers (543 nm) wird auf einen Durchmesser von etwa 12 mm aufgeweitet und anschließend mittels einer Lochmaske 12, in der eine Vielzahl äquidistanter Löcher ausgebildet sind, in eine entsprechende Anzahl paralleler Einzelstrahlen aufgeteilt. Gemäß Figur 2, verlaufen diese Einzelstrahlen, die nur schematisch durch punktierte Linien angedeutet sind, parallel zur optischen Achse A des Systems. Durch eine Aberroskoplinse 14 (Sammellinse) vor dem Auge 10 werden diese Strahlen so gebrochen, daß sie in einem bestimmten Abstand vor der Netzhaut 20 fokussiert werden (Fokus F). Bei einem rechtsichtigen Auge hat die Aberroskoplinse z. B. einen Brechwert von +4dpt. Im aberrationsfreien Idealauge entsteht auf diese Weise ein völlig unverzerrtes Lichtpunktmuster auf der Netzhaut 20. Die Pupille ist mit dem Bezugszeichen 18 angedeutet.

Weist das Auge 10 jedoch eine Aberration auf, so werden die Musterpunkte entsprechend den Abbildungsfehlern verschoben, da jeder Einzelstrahl nur einen ganz bestimmten Ort der Pupille 18 passiert und gemäß den irregulären optischen Wirkungen eine Abweichung vom idealen Verlauf erfährt. Diese Abweichung vom idealen Verlauf entspricht dem optischen Abbildungsfehler des gesamten optischen Systems des Auges 10 bezüglich eines Lichtstrahls, der den bestimmten Ort innerhalb der Pupille passiert. Auf der Hornhaut haben die Einzelstrahlen z. B. in x- und y-Richtung einen konstanten Abstand von 1,0 mm und ihr Durchmesser beträgt beispielhaft etwa 0,5 mm. Das gesamte parallele Meßstrahlbündel hat auf der Hornhaut z. B. eine Abmessung von 8 x 8 mm.

Mittels eines Halbspiegels 16 wird das auf der Netzhaut 20 erzeugte Lichtpunktmuster über eine Ophthalmoskoplinse 22 und ein Objektiv 24 für das Netzhautbild auf eine Sensorfläche 28 einer Festkörper-Bildkamera (CCD-Kamera) abgebildet, um das entstehende Lichtpunktmuster rechnerisch zu verarbeiten. Die Abweichungen der Orte der Lichtpunkte, bezogen auf die äquidistante, regelmäßige Struktur des fehlerfreien Auges, ergibt die Möglichkeit, die Wellenfrontaberration W (x, y) als Ortsfunktion über die Pupillenfläche des Auges zu ermitteln. Die Ortsfunktion kann mittels eines Satzes von Polynomen approximiert werden, z. B. Taylor-Polynomen oder Zernike-Polynomen. Die Zernike-Polynome werden hier bevorzugt, weil ihre Koeffizienten $K_i$ den Vorteil eines direkten Bezuges zu den Bildfehlern haben, wie Öffnungsfehler, Koma, Astigmatismus, Verzeichnung. Mit den Zernike-Polynomen $Z_i$ (x, y) läßt sich die Wellenfrontaberration W wie folgt darstellen:

$$W(x,y) = \Sigma_i\ C_i\ x\ Z_i(x,y).$$

Mit (x,y) sind die kartesischen Koordinaten in der Pupillenebene bezeichnet.

Mit der Bestimmung von z. B. den ersten 14 Koeffizienten $C_i$ (i = 1,2, ..., 14)der Zernike-Polynome ist eine hinreichend genaue Beschreibung der Wellenfrontaberration W(x,y) als Funktion der Ortskoordinaten der freien Pupillenfläche möglich. Auf diese Weise ergibt sich ein sog. Wellenfrontaberrationsgebirge, d. h. in einer dreidimensionalen Darstellung eine Funktion über den Ortskoordinaten x,y, die den jeweils lokalen Abbildungsfehler angibt. Außer den Zernike-Polynomen können auch andere Möglichkeiten gewählt werden, die Wellenfront mathematisch zu beschreiben, z. B. Taylor-Reihen. Die Zernike-Polynome sind nur das hier gewählte Ausführungsbeispiel.

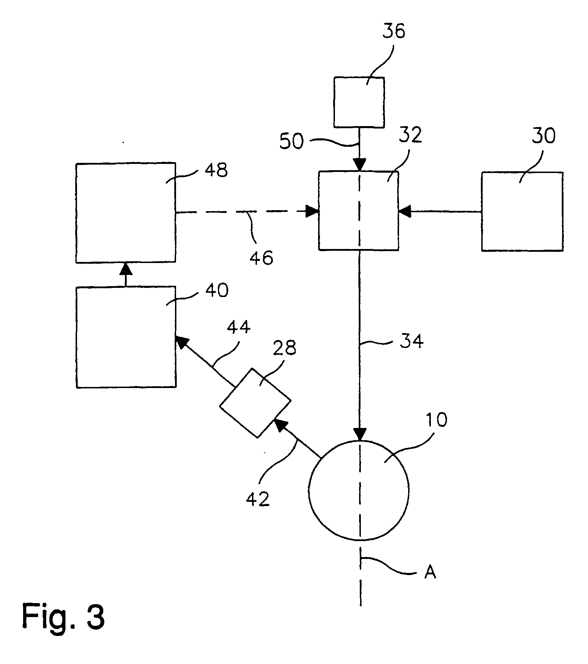

Aus dieser Wellenfrontaberration W(x,y) wird mittels eines Rechners 48 (Figur 3) ein sog. Fotoablationsprofil berechnet. Der Rechner ermittelt also letztlich aus dem Lichtpunktmuster die Wellenfrontaberration in Form einer bestimmten Anzahl von Zernike-Koeffizienten und dann aus der Wellenfrontaberration ein Fotoablationsprofil, d. h. Daten darüber, bis zu welcher Tiefe am jeweiligen Pupillenort die Hornhaut abgetragen (ablatiert) werden muß, um die Wellenfrontaberration zu verkleinern. Das Ablationsprofil, also die Schichtstärke des abzutragenden Materials in Abhängigkeit vom Ort (X-Y-Koordinaten) kann auf verschiedene Weise aus der Wellenfront (Aberration) bestimmt werden:

Grundsätzlich erfolgt die Berechnung des Ablationsprofils für ein zu korrigierendes Auge mit einem entsprechenden Augenmodell.

Dazu wird die Wellenfrontaberration auf die Hornhautoberfläche unter Berücksichtigung der geometrischen Eigenschaften des Auges, wie z. B. der Hornhautdicke, Abstand zwischen Hornhautrückfläche und Linsenvorderfläche, Abstand zwischen Linsenvorderfläche und Linsenrückfläche, Abstand zwischen Linsenrückfläche und Netzhaut, mathematisch projiziert. Weiterhin werden bei der Berechnung des Ablationsprofils die Brechungsindizes der einzelnen optischen Elemente des Auges berücksichtigt (z. B. Tränenfilm n=1,337, Hornhaut n=1,37, Kammerwasser n=1,337 usw.). Die Wellenfront beschreibt im wesentlichen die Laufzeitunterschiede des Lichts, d. h. die optische Wegstrecke. Dividiert man die optische Wegstrecke durch den Brechungsindex, so erhält man den geometrischen Weg. Es läßt sich somit aus der Projektion der Wellenfront auf die Hornhaut das zugehörige Ablationsprofil ableiten. In der Art einer Iteration wird an der gegebenen Stelle der Hornhaut eine Ablationstiefe (bei LASIK entsprechend eine Tiefe des im Stroma ablatierten Materials) mathematisch angenommen und berechnet, wie sich eine solche Ablation auf die Laufzeitunterschiede der Strahlen auswirken würde. Ziel ist eine Angleichung der Laufzeiten der Strahlen an allen Orten der Hornhaut derart, daß die Wellenfrontaberration möglichst gering wird. Dabei muß berücksichtigt werden, daß die Wellenfront auch Werte annehmen kann, die in ihrer physikalischen Bedeutung einen Auftrag von Gewebe bedeuten (d. h. eine Verstärkung der Hornhaut), was in der Regel nicht möglich ist. Deshalb muß das Ablationsprofil entsprechend angepaßt werden, d. h. insgesamt so verschoben werden, daß nur durch Ablation (Abtrag) von Gewebe das gewünschte Zielprofil der Hornhaut erreicht wird.

Die Wellenfrontaberration läßt sich nicht nur in der Pupillenebene (Eintrittspupille; englisch: entrance pupil) berechnen, sondern auch direkt an der Hornhaut. Unter Berücksichtigung der entsprechenden Brechungsindizes ergibt sich somit das eigentliche Ablationsprofil für einen bestimmten Pupillendurchmesser.

Gemäß der Erfindung wird eine Korrektur der für die Ermittlung des Ablationsprofils verwendeten Wellenfrontaberration W(x,y) dahingehend vorgenommen wird, daß der Heilungsprozeß des Auges nach der Operation mitberücksichtigt wird. Es hat sich nämlich herausgestellt, daß der Heilungsprozeß eine Änderung der optischen Eigenschaften des Auges zur Folge hat und daß zur Erzielung bester Ergebnisse diese Änderungen bei der zugrundegelegten Wellenfrontaberration berücksichtigt werden sollten. Dies geschieht wie folgt:

In die obige Gleichung, in der die Wellenfrontaberration $W(x,y)$ als Summe von Zernike-Polynomen $Z_i(x,y)$ dargestellt ist, werden sog. Korrekturfaktoren ("fudge factors") eingeführt:

$$W(x,y) = \sum_{i=0}^{n} A_i \times C_i \times Z_i(x,y)$$

Im Vergleich zur obigen Formel sind in der Summe von Zernike-Koeffizienten und Zernike-Polynomen jeweils Korrekturfaktoren $A_i$ hinzugefügt worden, die empirisch dem Wundheilungsprozeß Rechnung tragen. Mit anderen Worten: Die vorstehende Funktion $W(x,y)$ beschreibt die zu korrigierende Wellenfront am Auge unter Berücksichtigung von postoperativen Änderungen einzelner optischer Bildfehler (Zi) durch die Wundheilung. Dabei sind insbesondere klinisch relevant die Zernike-Koeffizienten von nullter bis achter Ordnung. Die Polynom-Koeffizienten Ci beschreiben, wie oben bereits erläutert ist, die Größe des Bildfehlers aus der beschriebenen Messung.

Es hat sich empirisch gezeigt, daß der klinisch relevante Wertebereich der Korrekturfaktoren $A_i$ im Bereich von -1000 bis 0 bis +1000 liegt. Es wurde weiter empirisch ermittelt, daß die klinischen Korrekturfaktoren $A_i$ für jeden Koeffizienten $C_i$ unterschiedliche Werte annehmen. $A_i$ ist also eine Funktion von $C_i$. Diese funktionale Abhängigkeit $A_i = f_i(C_i)$ ist unterschiedlich für die einzelnen Koeffizienten $C_i$, d. h. die Funktion $f_i$ hat verschiedene Verläufe für die einzelnen Koeffizienten $C_i$.

Es hat sich weiter gezeigt, daß die Funktion $a_i = f_i(C_i)$ weiterhin vom jeweils verwendeten therapeutischen Lasersystem abhängig ist, da der postoperative Heilungsverlauf vom jeweils verwendeten Lasersystem selbst abhängig ist. Dies bedeutet, es können in der Regel keine allgemein gültigen (abstrakten) Daten für die klinischen Korrekturfaktoren $A_i$ angegeben werden, vielmehr müssen diese Korrekturfaktoren empirisch (experimentell) klinisch für das jeweils verwendete Lasersystem ermittelt werden, wobei der oben angegebene typische Wertebereich von -1000 über 0 bis +1000 gilt, insbesondere für das hier verwendete Lasersystem mit dem Verkaufsnamen "Allegretto" der Firma Wave-Light, Erlangen, Deutschland.

Wie gesagt, können aufgrund der Wellenfrontaberration ermittelte Ablationsprofile, wenn die vorstehend genannten Korrekturfakten $A_i$ nicht verwendet werden, zu einer Überbewertung oder Unterbewertung einzelner Bildfehler aufgrund der Wundheilung nach dem refraktiven Eingriff führen, bei LASIK also u. a. das Anheilen des zurückgeklappten Scheibchens ("flap"). Z. B. muß für die Korrektur eines Komas von etwa $Z_7 = 0,3\ \mu m$ ein Koma von $Z_7 = 0,5\ \mu m$ von der Hornhaut abgetragen werden, damit nach dem Abschluß der Wundheilung (z. B. Epithelschluß, ca. 7 Tage) ein $Z_7 = 0$ resultiert ("Z" steht hier für den Zernike-Koeffizienten als Beispiel).

Die gemäß obiger Vorgabe ermittelten Korrekturfaktoren $A_i$ werden im Rechner abgelegt und das Computerprogramm arbeitete sie (automatisch) in das letztlich zur Anwendung kommende Ablationsprofil ein.

Alternativ zur vorstehend beschriebenen Berechnung des Ablationsprofils aus der Wellenfrontaberration kann das Ablationsprofil auch direkt aus einer Projektion von Punkten auf die Hornhaut und die Netzhaut berechnet werden. Fällt ein Lichtstrahl mit bekannten Einfallswinkeln und Koordinatenpunkten auf die Hornhaut und dann in das Auge, so wird dieser Lichtstrahl entsprechend den optischen Eigenschaften des Auges auf der Netzhaut abgebildet. Da die Position des Lichtstrahls auf der Hornhaut und die Einfallswinkel des Strahls bekannt sind, läßt sich durch Messung der Position des Lichtstrahls auf der Netzhaut der optische Strahlengang reproduzieren. Wird dabei festgestellt, daß die Position des Lichtstrahls auf der Netzhaut von der Sollposition abweicht (die Sollposition bedeutet eine aberrationsfreie Abbildung), so läßt sich aus der Positionsabweichung die Aberration ermitteln. Das Licht wird entsprechend der geometrischen Krümmung der Oberfläche der Hornhaut und den weiteren Aberrationsfehlern des Systems "Auge" gebrochen. Die vorstehend genannte Positionsabweichung des Lichtstrahls auf der Netzhaut kann durch eine entsprechende Änderung des Lichteinfallswinkels ausgedrückt werden. Der Lichteinfallswinkel ist proportional zur Ableitungsfunktion der Oberfläche der Hornhaut. Durch iteratives Vorgehen kann aus der Positionsverschiebung des Lichtstrahls auf der Netzhaut und der damit verbundenen Änderung des Lichteinfallswinkels auf eine (krankhafte) Änderung der Krümmung der Hornhautoberfläche geschlossen werden. Die Änderung der Krümmung der Hornhautoberfläche beschreibt also die Ableitungsfunktion des (gesuchten) Ablationsprofils. Wird dieses Verfahren mit einer ausreichenden Anzahl von Lichtstrahlen an unterschiedlichen Punkten des Auges durchgeführt (z. B. durch Projektion eines Gitters auf die Hornhaut), läßt sich die gesamte Ableitungsfunktion des (gesuchten) Ablationsprofils bestimmen. Hieraus kann dann mit bekannten mathematischen Verfahren (z. B. Spline-Interpolation und anschließende Integration) das Ablationsprofil berechnen.

Es hat sich gezeigt, daß Ablationsprofile, die mit Wellenfrontmessungen gewonnen worden sind, in einigen Fällen eine sog. Übergangszone erforderlich machen, weil ohne eine solche Übergangszone unter Umständen am Rand des Ablationsprofils ein bestimmter Rest an Material stehen bliebe, d. h. es würde sich auf der Hornhaut eine Stufe ergeben. Um eine derartige Stufe zu vermeiden, wird eine ca. 0,5 mm bis 3 mm breite Übergangszone um das Ablationsprofil herum nach außen hin vorgesehen, um eine glatte, stufenlose Fläche auf der gesamten Hornhaut zu gewährleisten.

Figur 3 zeigt schematisch das Rechner- und Steuersystem zur Durchführung einer Fotoablation gemäß dem errechneten Fotoablationsprofil. Die Fotoablation erfolgt sowohl oberflächlich auf der Hornhaut als auch intra-stromal.

Als Laser 30 für die Fotoablation kommt insbesondere in Betracht ein Excimerlaser (193 nm). Ebenfalls in Betracht kommen insbesondere Er:YAG-Festkörperlaser mit einer Wellenlänge von 2,94 µm und UV-Festkörperlaser (z.B. Nd:YAG mit 213 nm).

Die Laserstrahlung wird mittels eines galvanometrischen Abtasters (Scanner) 32 umgelenkt und der umgelenkte Laserstrahl 34 wird auf das Auge 10 gerichtet.

Koaxial mit dem Laserstrahl 34 wird ein weiterer Strahl einer sog. Positionierlichtquelle 36 auf das Auge 10 gerichtet. Der Strahl 50 der Positionierlichtquelle 36 definiert eine Bezugsachse A, die im Raum ortsfest ist.

Im Realfall bewegt sich das Auge 10 in Bezug auf die Achse A. Um bei derartigen Bewegungen den Bearbeitungsstrahl 34 und entsprechend das abzuarbeitende Ablationsprofil den Bewegungen des Auges anzupassen (nachzuführen) wird das Auge mit Infrarotstrahlung (nicht gezeigt) beleuchtet und mittels der CCD-Kamera 28 werden Bilder aufgenommen mit einer bestimmten Bildfolgefrequenz. Die Bildstrahlung 42 des Auges erzeugt also in der CCD-Kamera 28 Bilder, die elektronisch verarbeitet werden. Das elektronische Ausgangssignal 44 der Kamera 28 wird einer Bildverarbeitungseinrichtung 40 zugeführt und das Ergebnis der Bildverarbeitung wird in einen Rechner 48 eingegeben, der sowohl die Auswertung als auch die Steuerung des Scanners 32 übernimmt. Die Bildverarbeitung und die Positionierung des Auges sowie die Anpassung der Scannerbewegung und damit des Ablationsprofils an die momentane Position des Auges sind als solche bekannt (DE 197 02 335 C1). Der Rechner 48 gibt also ein entsprechendes Stellsignal 46 an den Scanner (Abtaster) 32, so daß der Laserstrahl 34 so gesteuert wird, daß in Bezug auf eine bestimmte Augenposition, in Bezug auf die auch die Wellenfrontablation gemessen worden ist, auch das Ablationsprofil abgearbeitet wird. Auf diese Weise können die optischen Fehler des gesamten Auges durch Fotoablation der Hornhaut korrigiert werden. Das hier im vorstehenden Sinne abgearbeitete Ablationsprofil ist das aus der Wellenfrontmessung gewonnene und um die oben erläuterten empirischen Korrekturfaktoren aufgrund der Wundheilung abgeänderte Ablationsprofil.

Beim vorstehend abgehandelten Ausführungsbeispiel wurde die Wellenfrontaberration mittels Gitterpunktverschiebung ermittelt (z. B. gemäß der Arbeit von J. Liang et al.). Es ist grundsätzlich möglich, die Wellenfrontaberration auch anders zu messen (z. B. gemäß der oben zitierten Arbeit von H. C. Howland und B. Howland) oder auch gemäß einer Arbeit von G. Smith, R. A. Applegate und H. C. Howland *Ophthal. Physiol. opt.* Vol. 16, No. 3, pp. 222-229, 1996 oder der Arbeit von G. Walsh, W. N. Charman und H. C. Howland in *Optical Society of America 1984*, S. 987-992.


**Patentansprüche**

1. Vorrichtung für die photorefraktive Hornhautchirurgie oder LASIK, des Auges zur Korrektur von Sehfehlern höherer Ordnung mit

   - einem Aberroskop (12, 14, 16, 22, 24, 28) zum Messen der Wellenfrontaberration (W(x, y)) gesamten optischen Systems des zu korrigierenden Auges in bezug auf eine bestimmte Augenposition,
   - Mitteln (48) zum Ableiten eines Photoablationsprofils aus der gemessenen Wellenfrontaberration derart, dass eine Photoablation gemäß dem Photoablationsprofil die Wellenfrontaberration des behandelten Auges minimiert, und
   - eine Laserstrahlungsquelle (30) und Mitteln (32, 38, 40, 48) zum Steuern der Laserstrahlung in bezug auf die bestimmte Augenposition entsprechend dem Photoablationsprofil,

   wobei
   die Mittel (48) zum Ableiten eines Photoablationsprofils mittels der gemessenen Wellenfrontaberration abgespeicherte Korrekturfaktoren (Ai) enthalten, die einer Änderung der optischen Eigenschaften des Auges im Heilungsprozess nach der Operation entsprechen und gemäß denen das mittels der gemessenen Wellenfrontaberration abgeleitete Photoablationsprofil geändert wird.

2. Vorrichtung nach Anspruch 1, **gekennzeichnet durch** eine Einrichtung (38, 40, 48) zum Ermitteln einer momentanen Augenposition und einer Einrichtung (48) zum Anpassen des Photoablationsprofils an die Augenposition.

3. Vorrichtung nach einem der Ansprüche 1 oder 2, **dadurch gekennzeichnet, dass** die Korrekturfaktoren wie folgt in eine ermittelte Wellenfrontaberration W(x,y) eingehen:

$$W(x,y) = \sum_{I=0}^{n} A_i \times C_i \times Z_i (x,y)$$

wobei $Z_i$ die Zernike-Polynome, $C_i$ die Polynom-Koeffizienten, $A_i$ die Korrekturfaktoren, i ein laufender Index von 0 bis n und n die Anzahl der Zernike-Polynome sind, die verwendet werden.

**Claims**

1. A device for photorefractive surgery on the cornea, or LASIK, of the eye for the correction of sight defects of a higher order, said device comprising

   - an aberroscope (12, 14, 16, 22, 24, 28) for measuring the wave-front aberration (W (x, y)) of the entire optical system of the eye to be corrected in relation to a specific eye position,
   - means (48) for deriving a photoablation profile from the measured wave-front aberration in such a way that a photoablation in accordance with the photoablation profile minimises the wave-front aberration of the treated eye, and
   - a laser radiation source (30) and means (32, 38, 40, 48) for controlling the laser radiation in relation to the specific eye position in accordance with the photoablation profile,

   wherein
   the means (48) for deriving a photoablation profile by means of the measured wave-front aberration contain stored correction factors (Ai) that correspond to a change of the optical properties of the eye during the post-operative healing process and according to which the photoablation profile derived by means of the measured wave-front aberration is changed.

2. The device according to claim 1, **characterised by** a device (38, 40, 48) for ascertaining an instantaneous eye position and a device (48) for adapting the photoablation profile to the eye position.

3. The device according to one of claims 1 or 2, **characterised in that** the correction factors enter into an ascertained wave-front aberration W (x, y) as follows:

$$W (x, y) = \sum_{i=0}^{n} A_i \times C_i \times Z_i (x, y)$$

wherein $Z_i$ are the Zernicke polynomials, $C_i$ are the polynomial coefficients, $A_i$ are the correction factors, i is a running index from 0 to n and n is the number of Zernicke polynomials which are used.

**Revendications**

1. Dispositif pour la chirurgie photoréfractive de la cornée ou LASIK, de l'oeil pour la correction des défauts de vision d'ordre éievé comprenant

   - un aberroscope (12, 14, 16, 22, 24, 28) destiné à mesurer l'aberration du front d'onde W(x, y) de l'ensemble du système optique de l'oeil à corriger par rapport à une position déterminée de l'oeil,
   - un moyen (48) pour déduire un profil de photo-ablation à partir de l'aberration de front d'onde mesurée de telle manière qu'une photo-ablation conforme au profil de photo-ablation réduise au minimum l'aberration de front d'onde de l'oeil traité, et

- une source de faisceau laser (30) et un moyen (32, 38, 40, 48) pour commander le rayonnement de laser par rapport à la position de l'oeil déterminée de façon correspondant au profil de photo-ablation,

dans lequel
le moyen (48) pour déduire un profil de photo-ablation au moyen de l'aberration de front d'onde mesurée contient des facteur de correction (Ai) enregistrés qui correspondent à une modification des propriétés optiques de l'oeil dans le processus de guérison après l'opération et en fonction desquels le profil de photo-ablation déduit de l'aberration de front d'onde mesurée est modifié.

2. Dispositif selon la revendication 1, **caractérisé par** un système (38, 40, 48) pour relever la une position momentanée de l'oeil et un système (48) pour adapter le profil de photo-ablation à la position de l'oeil.

3. Dispositif selon l'une des revendications 1 ou 2, **caractérisé en ce que** les facteurs de correction entrent comme suit dans une aberration de front d'onde W(x, y) :

$$W(x,y) = \sum_{i=0}^{n} A_i \times C_i \times Z_i(x,y)$$

où Zi sont les polynômes de Zernicke, Ci sont les coefficients de polynôme, Ai sont les facteurs de correction, i un indice variant de 0 à n et n est le nombre des polynômes de Zernicke qui sont utilisés.

Ideale Wellenfront (sphärisch),
bezogen auf F

Reale Wellenfront (aspärisch)

W = Wellenfront-Aberration

Fig. 1

Fig. 2

Fig. 3